# EUROPEAN PATENT SPECIFICATION

(11) **EP 1 578 412 B1**
(45) Date of publication and mention of the grant of the patent: **29.09.2010**
(21) Application number: 03814205.5
(22) Date of filing: 19.12.2003
(51) Int. Cl.: A61K 31/34, A61K 31/415, A61P 25/16

(54) **PHARMACEUTICAL COMPOSITIONS AND METHOD OF TREATING PARKINSON'S DISEASE**
PHARMAZEUTISCHE ZUSAMMENSETZUNGEN UND VERFAHREN ZUR BEHANDLUNG VON MORBUS PARKINSON
COMPOSITIONS PHARMACEUTIQUES ET MÉTHODE DE TRAITEMENT DE LA MALADIE DE PARKINSON

(30) Priority: 23.12.2002 US 436051 P; 20.02.2003 US 448833 P
(43) Date of publication of application: 28.09.2005
(73) Proprietor: Merck Frosst Company, Kirkland, Québec H9H 3L1 (CA); University of Medicine and Dentistry of New Jersey, Newark, NJ 07107-3001 (US)
(72) Inventor: HATHAWAY, Nancy, Kirkland, Québec H9H 3L1 (CA); MARK, Margery, Newark, NJ 07107-3001 (US)
(74) Representative: Buchan, Gavin MacNicol
(86) International application number: PCT/US2003/040561
(87) International publication number: WO 2004/058163

(56) References cited:
- WO-A-03/088958
- TEISMANN P ET AL: "PHARMACOLOGICAL INHIBITION OF COX - 2 PROVIDES NEUROPROTECTION IN THE MTPT-MOUSE MODEL OF PARKINSON'S DISEASE" ABSTRACTS OF THE SOCIETY FOR NEUROSCIENCE, SOCIETY FOR NEUROSCIENCE, WASHINGTON, DC, US, 1 January 2002 (2002-01-01), XP009104620 ISSN: 0190-5295
- TEISMANN PETER ET AL: "Inhibition of the cyclooxygenase isoenzymes COX-1 and COX-2 provide neuroprotection in the MPTP-mouse model of Parkinson's disease" SYNAPSE, WILEY AND SONS, CHICHESTER, GB, vol. 39, no. 2, 1 February 2001 (2001-02-01), pages 167-174, XP009104619 ISSN: 0887-4476
- DATABASE BIOSIS [Online] TEISMANN P. ET AL: 'Cyclooxygenase-2 deficient mice are resistant to MPTP', XP002979580 Database accession no. PREV200100562508 & SOCIETY FOR NEUROSCIENCE vol. 27, no. 2, 2001, page 1808

## Description

### FIELD OF THE INVENTION

This invention is directed to the use of certain pharmaceutical compositions for treating Parkinson's disease. In particular, this invention is directed to pharmaceutical compositions for treating Parkinson's disease comprising rofecoxib, with concomitant use of specified anti-Parkinson's drugs.

### RELATED BACKGROUND

Inhibitors of cyclooxygenase-2 are a sub-class of the class of drugs known as non-steroidal antiinflammatory drugs (NSAIDs). The NSAIDs are active in reducing the prostaglandin-induced pain and swelling associated with the inflammation process but are also active in affecting other prostaglandin-regulated processes not associated with the inflammation process. Thus, use of high doses of most common NSAIDs can produce severe side effects, including life threatening ulcers, that limit their therapeutic potential. An alternative to NSAIDs is the use of corticosteroids, which have even more drastic side effects, especially when long term therapy is involved.

Previous NSAIDs have been found to prevent the production of prostaglandin by inhibiting enzymes in the human arachidonic acid/prostaglandin pathway including the enzyme cyclooxygenase (COX). The recent discovery that there are two isoforms of the COX enzyme, the first, COX-1, being involved with physiological functions and the second, COX-2, being induced in inflamed tissue, has given rise to a new approach. While conventional NSAIDs block both the COX-1 and COX-2 enzymes, the identification of the inducible COX-2 enzyme and it's association with inflammation has provided a viable target of inhibition which more effectively reduces inflammation and produces fewer and less drastic side effects. A number of selective COX-2 inhibitors have been identified, including rofecoxib (VIOXX^{®}), etoricoxib (ARCOXIA™), celecoxib (CELEBREX®) and valdecoxib (BEXTRA™), and much research continues in this area.

Diseases of the extrapyramidal motor systems cause either a loss of movement (akinesia) accompanied by an increase in muscle tone (rigidity) or abnormal involuntary movements (dyskinesias) often accompanied by a reduction in muscle tone. The akinetic-rigid syndrome called parkinsonism, and dyskinesias represent opposite ends of the spectrum of movement disorders (for review see C. D. Marsden in Oxford Textbook of Medicine, 3rd Edition, Oxford University Press, 1996, vol. 3, pages 3998-4022).

In 50 to 80% of patients, Parkinson's disease begins insidiously with a resting 4-to 6-Hz pill-rolling tremor of one hand. The tremor is maximal at rest, diminishes during movement, and is absent during sleep; it is enhanced by emotional tension or fatigue. Usually, the hands, arms, and legs are most affected, in that order. Jaw, tongue, forehead, and eyelids may also be affected, but the voice escapes the tremor. In many patients, only rigidity occurs; tremor is absent. Rigidity progresses, and movement becomes slow (bradykinesia), decreased (hypokinesia), and difficult to initiate (akinesia). Rigidity and hypokinesia may contribute to muscular aches and sensations of fatigue. The face becomes masklike, with mouth open and diminished blinking, which may be confused with depression. The posture becomes stooped. Patients find it difficult to start walking; the gait becomes shuffling with short steps, and the arms are held flexed to the waist and do not swing with the stride. Steps may inadvertently quicken, and the patient may break into a run to keep from falling (festination). The tendency to fall forward (propulsion) or backward (retropulsion) when the center of gravity is displaced results from loss of postural reflexes. Speech becomes hypophonic, with a characteristic monotonous, stuttering dysarthria. Hypokinesia and impaired control of distal musculature results in micrographia and increasing difficulty with activities of daily living. Dementia affects at least 50% of patients, and depression is common.

During examination, passive movement of the limbs is met with a plastic, unvarying leadpipe rigidity; superimposed tremor bursts may have a ratchet-like cogwheel quality. The sensory examination is usually normal. Signs of autonomic nervous system dysfunction (eg, seborrhea, constipation, urinary hesitancy, orthostatic hypotension) may be found. Muscle strength is usually normal, although useful power may be diminished and the ability to perform rapid successive movements is impaired. Reflexes remain normal but may be difficult to elicit in the presence of marked tremor or rigidity.

Early signs, including infrequent blinking and lack of facial expression, decreased movement, impaired postural reflexes, and the characteristic gait abnormality, suggest the disease. Tremor occurs initially in about 70% of patients but often becomes less prominent as the disease progresses. Although rigidity is occasionally minimal or lacking, tremor without the above features suggests an alternate diagnosis or the need for a later reevaluation, because additional signs will develop if the patient has Parkinson's disease. Patients with essential tremor --the disorder most commonly confused with Parkinson's disease--have normal facies and rates of movement and no gait impairment. Furthermore, essential tremor is an action tremor rather than a resting tremor, which is most common in Parkinson's disease. Elderly persons with reduced spontaneity of movement, short-stepped (rheumatic) gait, and mild depression or dementia may be more difficult to distinguish from those with Parkinson's disease. Causes of the disease may be discerned from the history.

Several classes of drugs have been utilized in the treatment of Parkinson's disease with varying degrees of success. These classes include anticholinergic agents, including antihistamines, such as diphenhydramine (e.g. BENADRYL®) and orphenadrine (e.g. NORFLEX®), Antidepressants, such as amitriptyline (e.g. ELAVIL®), doxepin (e.g. SINEQUAN®), imipramine and nortriptyline; and miscellaneous anticholinergic agents such as benztropine (e.g. COGENTIN®), biperidin (e.g. AKINETON®), procyclidine, and trihexyphenidyl (e.g. ARTANE®). Antiparkinson drugs further include dopaminergic agents, including dopamine precusors (with decarboxylase), such as carbidopa/levodopa (e.g. SINEMET CR®); Dopamine receptor agonists, including bromocriptine (PARLODEL®), pergolide (e.g. PERMAX®), pramipexole (e.g.MIRAPEX®), carbergoline (DOSTINEX®) and ropinorole (e.g. REQUIP®). Antiparkinson drugs further include monoamine oxidase type B (MAO-B) inhibitors, including selegiline (e.g.ELDEPRYL®). These classes also include agents for which the mechanism of action is not fully understood, such as amantadine (e.g. SYMMETREL®) (most likely an NMDA receptor antagonist). Adjunctive treatment with levodopa and peripherally-acting catechol-O-methyltransferase (COMT) inhibitors such as entacapone (e.g. COMTAN®) and tolcapone (e.g. TASMAR®) is another option.

Levodopa, the metabolic precursor of dopamine, crosses the blood-brain barrier into the basal ganglia where it is decarboxylated to form dopamine, replacing the missing neurotransmitter. Bradykinesia and rigidity are helped most, although tremor is often substantially reduced. Mildly affected patients may return to nearly normal, and bedridden patients may become ambulatory. Co-administration of the peripheral decarboxylase inhibitor carbidopa lowers dosage requirements by preventing levodopa catabolism, thus decreasing side effects (nausea, palpitations, flushing) and allowing more efficient delivery of levodopa to the brain. Carbidopa/levodopa is available in fixed-ratio preparations of 10/100, 25/100, 25/250, and, in a controlled-release tablets, of 25/100 and 50/200 mg.

Treatment is often begun with one 25/100-mg tablet tid. Dosage is gradually increased as needed according to patient tolerance until maximum benefit is reached. Side effects may be minimized by gradually and cautiously increasing the dosage and by giving the drug with or after meals (although large amounts of protein may interfere with absorption of levodopa). Most moderate-to-severe patients require 400 to 1000 mg/day of levodopa in divided doses q 2 to 5 h with at least 100 mg/day of carbidopa to minimize peripheral side effects. Some patients may require up to 2000 mg/day of levodopa with at least 200 mg of carbidopa.

Involuntary movements (dyskinesias) in the form of orofacial or limb chorea or dystonia, a side effect of levodopa, often limit the dose. They tend to occur at lower doses as treatment continues. In some patients, the drug cannot reduce parkinsonism without producing some degree of dyskinesia. After 2 to 5 yr of treatment, > 50% of patients begin to experience fluctuations in their response to levodopa (wearing off or, less commonly, on-off effect). The duration of improvement after each dose of drug shortens, and superimposition of dyskinetic movements results in swings from intense akinesia to uncontrollable hyperactivity. Traditionally, such swings are managed by keeping individual doses of levodopa as low as possible and using dosing intervals as short as q 1 to 2 h. Dopamine-agonist drugs, controlled-release levodopa/carbidopa, or selegiline may be useful adjuncts. Other side effects of levodopa include orthostatic hypotension, nightmares, hallucinations, and, occasionally, toxic delirium. Hallucinations and delirium are most common in elderly, demented patients.

Some authorities believe that early levodopa therapy hastens the advent of problems (eg, dyskinesias, the on-off effect) and prefer to withhold levodopa as long as possible, relying on early use of dopamine agonists. Others regard these phenomena as part of the course of the disease and start levodopa with carbidopa early to obtain maximal improvement in the quality of life.

Amantadine 100 to 300 mg/day po is useful in treating early, mild parkinsonism for 50% of patients and in augmenting the effects of levodopa later in the disease. Its mechanism of action is uncertain; it may act by augmenting dopaminergic activity, anticholinergic effects, or both, but more recently has been shown to have activity at NMDA receptors. It has also recently been shown to be effective in reducing dyskinesias while augmenting antiparkinson effect in fluctuating patients. Amantadine often loses its effectiveness after a period of months when used alone. Side effects include lower extremity edema, livedo reticularis, and confusion.

Bromocriptine and pergolide are ergot alkaloids that directly activate dopamine receptors in the basal ganglia; pramipexole and ropinirole are non-ergot dopamine agonists that are more specific for the D₂ receptor. Bromocriptine 5 to 60 mg/day was the first agonist marketed for Parkinson's disease but is the least potent of the four drugs and is used infrequently now. Pergolide 0.1 to 5.0 mg/day, pramipexole 0.5 to 4.5 mg/day, and ropinirole 0.75 to 24 mg/day po are useful at all stages of the disease, particularly in later stages when response to levodopa diminishes or on-off effects are prominent. Use is often limited by a high incidence of adverse effects, including nausea, orthostatic hypotension, confusion; delirium, and psychosis. Adverse effects may be controlled by reducing the dose of levodopa. Using dopamine agonists early in treatment may delay the emergence of drug-induced involuntary movements and on-off effects, but this effect is unproved. This benefit may be due to their long half-lives: The prolonged dopamine receptor stimulation by these drugs is more physiologic than that by levodopa (which has a short plasma half-life), so the integrity of postsynaptic dopamine receptors is preserved and drug response is more normal. As the disease progresses, however, the agonists need to be supplemented with levodopa for adequate antiparkinsonian effect.

Selegiline, a monoamine oxidase type B (MAO-B) inhibitor, inhibits one of the two major enzymes that breaks down dopamine in the brain, thereby prolonging the action of individual doses of levodopa. At doses of 5 to 10 mg/day po, it does not cause hypertensive crisis (tyramine or cheese effect), common with nonselective MAO inhibitors, which block the A and B isoenzymes. In some patients with mild on-off problems, selegiline helps diminish the end-of-dose wearing off of levodopa's effect. Although virtually free of side effects (except for insomnia if given late in the day), selegiline can potentiate the dyskinesias, mental adverse effects, and nausea produced by levodopa, and the dose of levodopa may have to be reduced. Selegiline, used as initial treatment, can delay the initiation of levodopa by about 1 yr. Selegiline may potentiate residual dopamine in the brain of patients with early Parkinson's disease or reduce oxidative metabolism of dopamine in the brain, slowing the neurodegenerative process, but this mechanism is highly speculative.

Although used infrequently these days, anticholinergic drugs are may be used alone in the early stages of treatment and later to supplement levodopa. Commonly used anticholinergics include benztropine 0.5 to 2 mg po tid and trihexyphenidyl 2 to 5 mg po tid, and antihistamines with anticholinergic action (eg, diphenhydramine 25 to 200 mg/day po and orphenadrine 50 to 200 mg/day po); these drugs are only useful for treating tremor. Anticholinergic tricyclic antidepressants (eg, amitriptyline 10 to 150 mg po at bedtime) often are useful as adjuvants to levodopa, as well as in treating depression and secondary insomnia. Initially, the dose should be small, then increased as tolerated. Adverse effects include dry mouth, urinary retention, constipation, and blurred vision. Particularly troublesome in older patients are confusion, delirium, and impaired thermoregulation due to decreased sweating.

Catechol *O-*methyltransferase (COMT) inhibitors, such as tolcapone and entacapone, inhibit the peripheral (intestinal) breakdown of dopamine and therefore are useful as adjuncts to levodopa; they are never used without levodopa.

Propranolol 10 mg bid to 40 mg po qid occasionally helps when parkinsonian tremor is accentuated rather than quieted by activity or intention.

Table 1 is illustrative of the doses that have been found to be of therapeutic value in treating Parkinson's disease:

**TABLE 1**

| Agent | Dose (mg/day) po |
|---|---|
| Diphenhydramine | 25 to 200 |
| Orphenadrine | 50 to 200 |
| Amitriptyline | 10 to 150 |
| Doxepin | 10 to 150 |
| Imipramine | 10 to 150 |
| Nortriptyline | 10 to 150 |
| Benztropine | 0.5 to 6 |
| Biperiden | 2 to 6 |
| Ethopropazine | 40 to 400 |
| Procyclidine | 5 to 40 |
| Trihexyphenidyl | 2 to 15 |
| Carbidopa/levodopa | 75/300 to 250/2500 |
| Bromocriptine | 5 to 60 |
| Pergolide | 0.1 to 7 |
| Selegiline | 5 to 10 |
| Amantadine | 100 to 300 |

Further information on the use of Therapeutic was recently reviewed in The Lancet:

**TABLE 2**

| Therapeutic Interventions for signs and symptoms of Parkinson's disease according to main mechanism of action* ± | | | | | |
|---|---|---|---|---|---|
| | | | | | |
| **Drugs** | **Promote dopamine synthesis** | **Active specific receptors** | | **Prolong dopamine availability** | **Prolong Levodopa bioavailability** |
| Dopaminergic | *Levodopa+ dopa-decarboxylase inhibitor* | *Dopamine agonists* | | *MAO-B Inhibitors* | *COMT Inhibitors* |
| | | Apomorphine† | | Selegiline | Entacapone |
| | | Bromocriptine | | | Toicapone‡ |
| | | Cabergoline | | | |
| | | Dihydroergocriptine | | | |
| | | Lisuride | | | |
| | | Pergolide | | | |
| | | Piribedil | | | |
| | | Pramipexole | | | |
| | | Ropinirole | | | |
| Antiglutamaterigic | | | Amantadine§ | | |
| Anticholinergic | | | Benztropine | | |
| | | | Biperiden | | |
| | | | Orphenadrine | | |
| | | | Procyclidine | | |
| | | | Trihexyphenidyl | | |

| **Surgery** | **Lesion** | **Deep brain stimulation** | | **Transplantation** | |
|---|---|---|---|---|---|
| | Thalamotomy | Thalamus | | Fetal mesencephalic cells | |
| | Pallidomy | Pallidum | | | |
| | Subthalamic nucleotomy | Subthalamic nucleus | | | |
| | | | | | |
| **Rehabilitation procedures** | | | | | |
| | | | Physical therapy | | |
| | | | Occupational therapy | | |
| | | | Speech therapy | | |

| | | | | | |
|---|---|---|---|---|---|
| MAO-B-monoamine oxidase-B, COMT=catechol-O-methyltransferase. Therapeutic interventions for comorbidities are those of the comorbidities; they are not specific to Parkinson's disease. †not available for oral administration. ‡Use restricted or suspended in many countries due to hepatoxocity. Mechanism of action not fully known; the antiglutamatergic action is just part of a more complex effect. *THE LANCET. Vol. 359·May 4, 2002 | | | | | |

Treatment of akinetic-rigid conditions such as parkinsonism typically involves the use of levodopa, anticholinergics or dopamine agonists. Levodopa is converted into dopamine in the brain by the enzyme dopa decarboxylase. However, this enzyme is also present in the gut wall, liver, kidney and cerebral capillaries, thus the peripheral formation of levodopa metabolites may give rise to side-effects such as nausea, vomiting, cardiac dysrhythmias and postural hypotension. This peripheral decarboxylation is largely prevented by the addition of a selective extracerebral decarboxylase inhibitor, such as carbidopa or benserazide, which themselves do not penetrate the brain. Levodopa combined with carbidopa (SINEMET^{™}) or benserazide (MADOPAR^{™}) is now the treatment of choice when levodopa is indicated. Even then, this combination therapy may be associated with side effects such as dyskinesias and psychiatric disturbances.

An anticholinergic such as benzhexol or orphenadrine may be used, however, anticholinergics cause peripheral parasympathetic blockade which may cause dry mouth, blurred vision and constipation, and they may also precipitate glaucoma, urinary retention and a toxic confusional state.

Dopamine agonists such as bromocriptine (PARLODEL^{™}), lisuride, pergolide (CELANCE^{™}), pramipexole (MIRAPEX^{™}) and ropinirole (REQUIP^{™}) act directly on dopamine receptors and have a similar side-effect profile to levodopa.

Teismann et al, in Synapse 39:167-174 (2001), and in 'Pharmacological Inhibition of COX-2 provides Neuroprotection in the mPTP-Mouse Model of Parkinson's Disease', Abstracts of the Society for Neuroscience, January 2002, report that treatment with COX-2 inhibitors is effective in animal models of Parkinson's disease.

WO 03/088958 (which is prior art under Art 54(3) EPC) discloses the use of COX-2 inhibitors in combination with conventional anti-Parkinson's drugs.

In view of the short-comings of existing therapy, however, there is a need for new, safe and effective treatment for Parkinson's disease.

### SUMMARY OF THE INVENTION

This invention is directed to the use of certain pharmaceutical compositions for treating Parkinson's disease. In particular, this invention is directed to pharmaceutical compositions for treating Parkinson's disease comprising the use of the selective cyclooxygenase-2 inhibitor rofecoxib, with concomitant use of specified antiparkinson drugs.

### DETAILED DESCRIPTION OF THE INVENTION

The present invention provides a combination of pergolide, selegiline and rofecoxib for use in treatment of Parkinson's disease.

For purposes of this application, the terms "selective COX-2 inhibitor", "cyclooxygenase-2 selective inhibitor" and the like, refers to rofecoxib, and "antiparkinson agent" or "anti-Parkinson's drug" to a combination of pergolide and selegiline.

Whilst it is envisaged that a selective COX-2 inhibitor will be useful alone in the treatment of Parkinson's disease, it will be appreciated that a combination of a conventional antiparkinsonian drug and a selective COX-2 inhibitor will provide an enhanced effect over the use of antiparkinson medication, considered alone. Not only will such a combination result in improved efficacy at any selected dose of the anti-Parkinson's drug, the use of a selective COX-2 inhibitor will enable the use of a lower dose of the antiparkinsonian agent without compromising the efficacy of the antiparkinsonian agent, thereby minimizing the risk of adverse side-effects.

In a further or alternative aspect of the present invention, there is therefore provided a product comprising a selective COX-2 inhibitor and an antiparkinson agent as a combined preparation for simultaneous, separate or sequential use in the treatment or prevention of Parkinson's disease.

It will be appreciated that when using a combination of the present invention, the selective COX-2 inhibitor and the antiparkinson agent may be in the same pharmaceutically acceptable carrier and therefore administered simultaneously. They may be in separate pharmaceutical carriers such as conventional oral dosage forms which are taken simultaneously. The term "combination" also refers to the case where the compounds are provided in separate dosage forms and are administered sequentially. Therefore, by way of example, the antiparkinson agent may be administered as a tablet and then, within a reasonable period of time, the selective COX-2 inhibitor may be administered either as an oral dosage form such as a tablet or a fast-dissolving oral dosage form. By a "fast-dissolving oral formulation" is meant, an oral delivery form which when placed on the tongue of a patient, dissolves within about 10 seconds.

As used herein, the term "treatment" refers both to the treatment and to the prevention or prophylactic therapy of the aforementioned conditions.

The selective COX-2 inhibitors for use in the present invention is rofecoxib.

Suitable pharmaceutically acceptable salts of the selective COX-2 inhibitor of use in the present invention include acid addition salts which may, for example, be formed by mixing a solution of the compound with a solution of a pharmaceutically acceptable non-toxic acid such as hydrochloric acid, fumaric acid, maleic acid, succinic acid, acetic acid, citric acid, tartaric acid, carbonic acid, phosphoric acid or sulfuric acid. Salts of amine groups may also comprise the quaternary ammonium salts in which the amino nitrogen atom carries an alkyl, alkenyl, alkynyl or aralkyl group. Where the compound carries an acidic group, for example a carboxylic acid group, the present invention also contemplates salts thereof, preferably non-toxic pharmaceutically acceptable salts thereof, such as the sodium, potassium and calcium salts thereof.

Suitable pharmaceutically acceptable salts of the antiparkinson agents used in combination with a selective COX-2 inhibitor according to the present invention include those salts described above in relation to the salts of selective COX-2 inhibitor.

Preferably the compositions containing a selective COX-2 inhibitor of use according to the present invention are in unit dosage forms such as tablets, pills, capsules, wafers and the like. Additionally, the selective COX-2 inhibitors of use according to the present invention may be presented as granules or powders for extemporaneous formulation as volume defined solutions or suspensions. Alternatively, the selective COX-2 inhibitors of use according to the present invention may be presented in ready-prepared volume defined solutions or suspensions. Preferred forms are tablets and capsules.

For preparing solid compositions such as tablets, the principal active ingredient is mixed with a pharmaceutical carrier, e.g. conventional tableting ingredients such as corn starch, lactose, sucrose, sorbitol, talc, stearic acid, magnesium stearate, dicalcium phosphate or gums, and other pharmaceutical diluents, e.g. water, to form a solid preformulation composition containing a homogeneous mixture of a compound of the present invention, or a non-toxic pharmaceutically acceptable salt thereof. When referring to these pre-formulation compositions as homogeneous, it is meant that the active ingredient is dispersed evenly throughout the composition so that the composition may be readily subdivided into equally effective unit dosage forms such as tablets, pills and capsules. This solid preformulation composition is then subdivided into unit dosage forms of the type described above containing from 0.1 to about 500 mg of the active ingredient of the present invention. The tablets or pills of the novel composition can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permits the inner component to pass intact into the duodenum or to be delayed in release. A variety of materials can be used for such enteric layers or coatings, such materials including a number of polymeric acids and mixtures of polymeric acids with such materials as shellac, cetyl alcohol and cellulose acetate.

The liquid forms in which the novel compositions of the present invention may be incorporated for administration orally include aqueous solutions, suitably flavoured syrups, aqueous or oil suspensions, and flavoured emulsions with edible oils such as cottonseed oil, sesame oil, coconut oil, peanut oil or soybean oil, as well as elixirs and similar pharmaceutical vehicles. Suitable dispersing or suspending agents for aqueous suspensions include synthetic and natural gums such as tragacanth, acacia, alginate, dextran, sodium carboxymethylcellulose, methylcellulose, polyvinyl-pyrrolidone or gelatin.

Compositions of the present invention may also be administered via the buccal cavity using conventional technology, for example, absorption wafers.

Compositions in the form of tablets, pills, capsules or wafers for oral administration are particularly preferred.

A minimum dosage level for the selective COX-2 inhibitor is about 1mg per day, preferably about 5mg per day and especially about 10mg per day. A maximum dosage level for the selective COX-2 inhibitor is about 1500mg per day, preferably about 1000mg per day and especially about 500mg per day. The compounds are administered one to three times daily, preferably once or twice a day, and especially once a day.

It will be appreciated that the amount of the selective COX-2 inhibitor required for use in the treatment or prevention of Parkinson's disease will vary not only with the particular compounds or compositions selected but also with the route of administration, the nature of the condition being treated, and the age and condition of the patient, and will ultimately be at the discretion of the patient's physician or pharmacist.

### EXAMPLE 1

Patent 1, a 51 year old woman, was administered Part III (motor examination) of the Unified Parkinson's Disease Rating Scale (hereinunder abbreviated as UPDRS) and diagnosed as having Parkinson's disease (scoring a 20 in the test). After a period of evaluation of drug therapy options, the patient was prescribed pergolide, 0.25mg tid and selegiline 5mg po. After six (6) months on drug therapy, the patient was once again examined with the UPDRS motor subset and scored 16.5. After three (3) months on the drug, 25mg of VIOXX, once a day was added to the treatment regime. After four (4) additional months, the patient was once again administered the UPDRS, this time scoring a 6.0. After six (6) months on the VIOXX tripartite therapy, the patient was again administered the UPDRS motor examination and scored 4.0. (*see* Fahn S, Elton RL, Members of the UPDRS Development Committee. Unified Parkinson's disease rating scale. In: Fahn S, Marsden CD, Calne D, Goldstein M, eds. Recent developments in Parkinson's disease, Vol II. Florham Park, NJ: Macmillan Healthcare Information, 1987:153-163, 293-304):

## Claims

1. A combination of pergolide, selegiline and rofecoxib for use in treatment of Parkinson's disease.

2. A combination according to claim 1 wherein said treatment comprises administration of 0.25mg pergolide tid, 5mg selegiline and 25mg rofecoxib once a day.

## Patentansprüche

1. Eine Kombination aus Pergolid, Selegilin und Rofecoxib zur Verwendung bei der Behandlung von Parkinson-Krankheit.

2. Eine Kombination gemäß Anspruch 1, wobei die Behandlung die Verabreichung von 0,25 mg Pergolid t.i.d., 5 mg Selegilin und 25 mg Rofecoxib einmal am Tag umfasst.

## Revendications

1. Combinaison de pergolide, de sélégiline et de rofécoxib à utiliser dans le traitement de la maladie de Parkinson.

2. Combinaison selon la revendication 1, où le traitement comprend l'administration de 0,25 mg de pergolide trois fois par jour, de 5 mg de sélégiline et de 25 mg de rofécoxib une fois par jour.
